# EUROPEAN PATENT APPLICATION

(11) **EP 2 664 267 A1**
(43) Date of publication of application: **20.11.2013**
(21) Application number: 12775965.2
(22) Date of filing: 28.03.2012
(51) Int. Cl.: A61B 1/00, G02B 23/24

(54) **FLEXIBLE TUBE PART FOR ENDOSCOPE AND ENDOSCOPE HAVING FLEXIBLE TUBE PART**

(30) Priority: 28.04.2011 JP 2011101465
(71) Applicant: Olympus Corporation, Shibuya-ku Tokyo 151-0072 (JP)
(72) Inventor: IEDE, Taro, Tokyo 192-8512 (JP)
(74) Representative: Winter, Brandl, Fürniss, Hübner, Röss, Kaiser, Polte - Partnerschaft
(86) International application number: PCT/JP2012/058161
(87) International publication number: WO 2012/147443

(57) **Abstract**

A flexible tube portion (25) includes a spiral tube (81), a first connecting tube (111) which is arranged on a distal end portion (81a) side of the spiral tube (81) and a second connecting tube (121) which is arranged on a proximal end portion (81b) side of the spiral tube (81). The spiral tube (81) is movable along an axial direction of the spiral tube (81) with respect to at least one of the first connecting tube (111) and the second connecting tube (121).

## Description

### Technical Field

The present invention relates to a flexible tube portion of an endoscope having flexibility and an endoscope having this flexible tube portion.

### Background Art

In general, an endoscope has a flexible tube portion that is inserted into, for example, a sigmoid colon in a large intestine while bending. For example, Patent Literature 1 discloses such a flexible tube portion. This flexible tube portion has, for example, a spiral tube, a mesh tube that is arranged on the outer side of this spiral tube and laminated on the spiral tube, and an envelope that is arranged on the outer side of this mesh tube and laminated on the mesh tube. The mesh tube covers the spiral tube, and the envelope covers the mesh tube. In this manner, the flexible tube portion has a three-layer configuration.

Further, for example, in Patent Literature 2, a flexible tube portion has a front connecting tube which is arranged at a distal end portion of the flexible tube portion to couple a bend portion of an endoscope with the distal end portion of the flexible tube portion and a rear connecting tube which is arranged at a proximal end portion of the flexible tube portion to couple the proximal end portion of the flexible tube portion with an operating portion of the endoscope. The front connecting tube is coupled with a proximal end portion of the bend portion, and the rear coupling tube is coupled with the operating portion of the endoscope.

At this time, a distal end portion of a spiral tube, a distal end portion of a mesh tube, and a distal end portion of an envelope are fixed to the front connecting tube, respectively. Furthermore, a proximal end portion of the spiral tube, a proximal end portion of the mesh tube, and a proximal end portion of the envelope are fixed to the rear connecting tube.

Since the distal end portion of the spiral tube is fixed to the front connecting tube and the proximal end portion of the spiral tube is fixed to the rear connecting tube, a position of the spiral tube is regulated without moving along a longitudinal direction of the flexible tube portion. Therefore, in the longitudinal direction of the flexible tube portion, the spiral tube is arranged over the entire flexible tube portion, thereby avoiding crush of the entire flexible tube portion and local crush of the flexible tube portion.

### Citation List

### Patent Literatures

Patent Literature 1: Jpn. Pat. Appln. KOKAI Publication No. 2002-263059
Patent Literature 2: Jpn. Pat. Appln. KOKAI Publication No. 2009-153714

### Disclosure of Invention

In general, the sigmoid colon has a very small radius of curvature. Therefore, as described above, when the flexible tube portion is inserted into the sigmoid colon while bending, the flexible tube portion must also bend small in accordance with the sigmoid colon. When the flexible tube portion bends, generally, the inner side of the flexible tube portion is narrower (contracts) than a natural length (a linear state), and the outer side of the flexible tube portion is wider (extends) than the natural length (the linear state). This point can be likewise applied to the spiral tube.

However, as described above, to avoid the crush of the flexible tube portion, both end portions of the spiral tube are fixed, and a position of the spiral tube is regulated without moving along the longitudinal direction of the flexible tube portion. Therefore, the flexible tube portion (the spiral tube) hardly bends when the radius of curvature of the sigmoid colon becomes small, and large force is required to bend the flexible tube portion. In this manner, as the radius of curvature of the sigmoid colon is reduced, operability of the flexible tube portion is decreased.

Further, in case of inserting the flexible tube portion into the sigmoid colon or the like while bending, if the flexible tube portion does not bend flexibly in accordance with the sigmoid colon, the burden on the patient increases.

In view of the above-described problem, it is an object of the present invention to provide a flexible tube portion of an endoscope which does not require large force to bend the flexible tube portion at the time of bending the flexible tube portion with a small radius of curvature, has good operability, and can reduce the burden on the patient, and to provide an endoscope having this flexible tube portion.

One aspect of a flexible tube portion of an endoscope according to the present invention comprises: a spiral tube; a first connecting tube which is arranged on a distal end portion side of the spiral tube and connected to a node ring of a bend portion in the endoscope; and a second connecting tube which is arranged on a proximal end portion side of the spiral tube and connected to an operating portion of the endoscope, wherein the spiral tube is movable with respect to at least one of the first connecting tube and the second connecting tube in an axial direction of the spiral tube.

One aspect of the present invention provides an endoscope having the above-described flexible tube portion of an endoscope.

### Brief Description of Drawings

FIG. 1 is a schematic view of an endoscope according to the present invention;
FIG. 2A is a view showing a three-layer configuration of a flexible tube portion in a first embodiment;
FIG. 2B is an enlarged view of part of a mesh tube;
FIG. 3A is a view showing a state that a spiral tube bends in a state that both end portions of the spiral tube are fixed to a connecting tube;
FIG. 3B is a view showing a state that the spiral tube bends in a state that both the end portions of the spiral tube can slide in a mesh tube arranged in the connecting tube;
FIG. 3C is a view showing a state that the spiral tube bends in a state that both the end portions of the spiral tube can slide in the mesh tube arranged in the connecting tube;
FIG. 3D is a view showing a state that the spiral tube bends in a state that both the end portions of the spiral tube can slide in the connecting tube;
FIG. 4A is a view showing a configuration of a spiral tube in a second embodiment;
FIG. 4B is a view showing a first modification of the second embodiment, which is a configuration of a distal end portion side of a spiral tube in the first modification;
FIG. 4C is a view showing a second modification of the second embodiment, which is a configuration of a spiral tube in the second modification;
FIG. 4D is a view showing a third modification of the second embodiment, which is a configuration of a distal end portion side of a spiral tube in a third modification;
FIG. 4E is a view showing a fourth modification of the second embodiment, which is a configuration of a distal end portion side of a spiral tube in the fourth modification;
FIG. 4F is a view showing a state that an elastic member stretches when the spiral tube bends in the fourth modification; and
FIG. 5 is a view showing a configuration of a distal end portion side of a flexible tube portion in a third embodiment.

### Best Mode for Carrying Out the Invention

Embodiments according to the present invention will now be described hereinafter in detail with reference to the drawings.

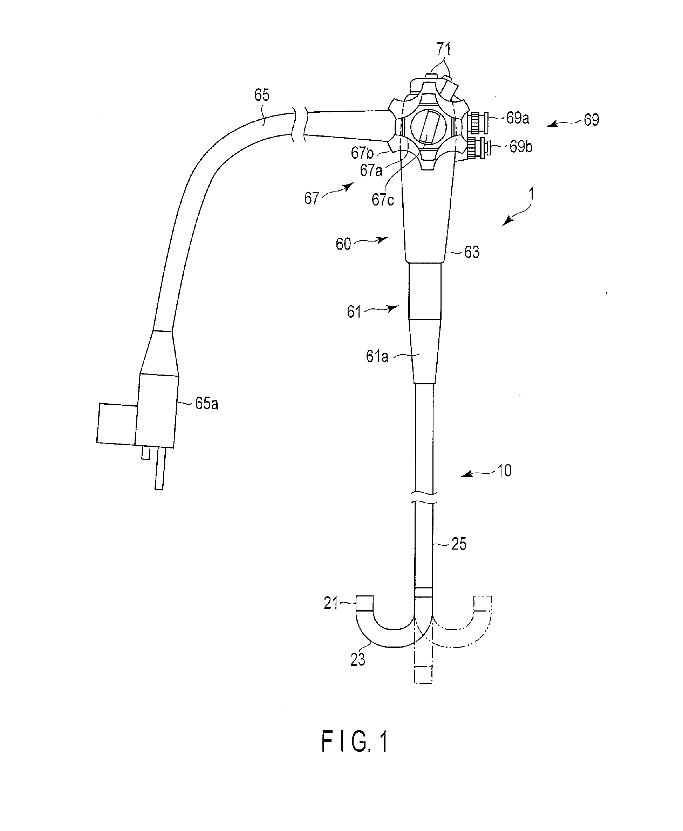

A first embodiment will now be described with reference to FIG. 1, FIG. 2A, FIG. 2B, FIG. 3A, FIG. 3B, and FIG. 3C.

As shown in FIG. 1, an endoscope 1 has an elongated insertion portion 10 which is inserted into a body cavity or the like of a patient and an operating portion 60 which is coupled with a proximal end portion of the insertion portion 10 and operates the endoscope 1.

The insertion portion 10 has a distal end hard portion 21, a bend portion 23, and a flexible tube portion 25 from a distal end portion side of the insertion portion 10 toward the proximal end portion side of the insertion portion 10. A proximal end portion of the distal end hard portion 21 is coupled with a distal end portion of the bend portion 23, and a proximal end portion of the bend portion 23 is coupled with the distal end portion of the flexible tube portion 25.

The distal end hard portion 21 is a distal end portion of the insertion portion 10, and it is hard.

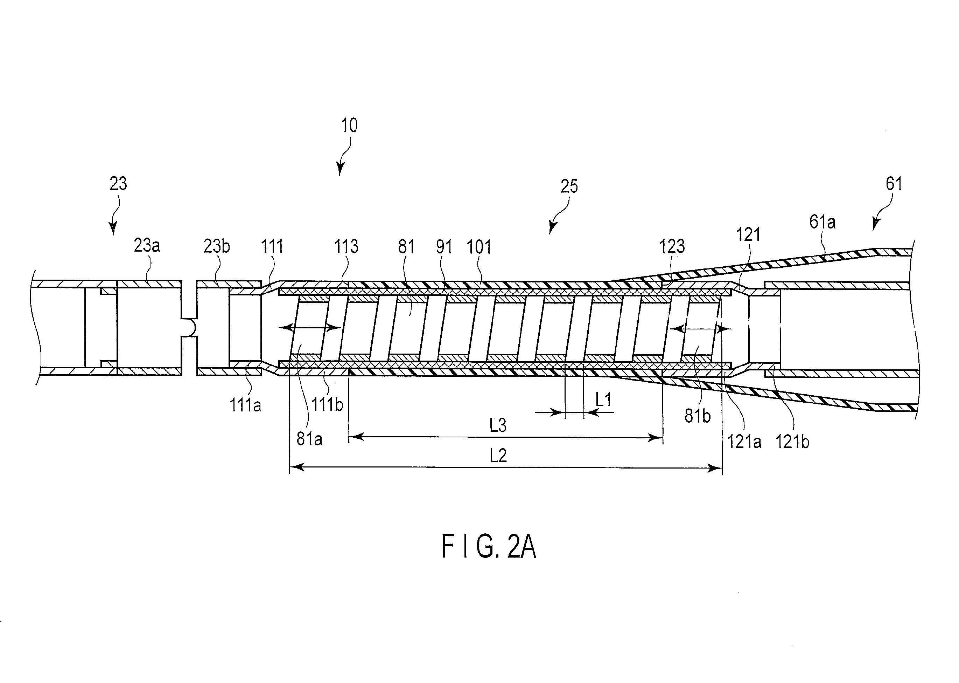

The bend portion 23 bends in a desired direction, for example, each of upper, lower, left, and right directions by an operation of a later-described bend operating portion 67. When the bend portion 23 bends, a position and a direction of the distal end hard portion 21 vary, an observation target is captured in an observation viewing field, and the observation target is illuminated with illumination light. The bend portion 23 is configured by coupling node rings 23a and 23b to allow their revolving motion along a longitudinal direction of the insertion portion 10 as shown in FIG. 2A. Each of the node rings 23a and 23b is covered with a non-illustrated mesh tube, and the non-illustrated mesh tube is covered with a non-illustrated envelope made of, for example, a resin or rubber.

The flexible tube portion 25 has desired flexibility and bends by external force. The flexible tube portion 25 is a tubular member extending from a later-described main body portion 61 in the operating portion 60. A configuration of the flexible tube portion 25 will be described later.

The operating portion 60 has a main body portion 61 from which the flexible tube portion 25 extends, a grip portion 63 which is coupled with a proximal end portion of the main body portion 61 and is gripped by an operator who operates the endoscope 1, and a universal cord 65 connected with the grip portion 63.

The main body portion 61 has an anti-folding portion 61a which is an exterior body of the main body portion 61.

The grip portion 63 has the bend operating portion 67 which bends the bend portion 23. The bend operating portion 67 has a left-and-right bend operation knob 67a which bends the bend portion 23 to left and right sides, an up-and-down bend operation knob 67b which bends the bend portion 23 upward and downward, and a fixing knob 67c which fixes a position of the bent bend portion 23.

Further, the grip portion 63 has a switch portion 69 which is operated by a hand of an operator when the grip portion 63 is gripped by the operator. The switch portion 69 has a suction switch 69a and an air supply/water supply switch 69b. The suction switch 69a is operated when the endoscope 1 sucks mucus or a fluid from a non-illustrated suction opening portion arranged in the distal end hard portion 21 through a non-illustrated suction channel. The air supply/water supply switch 69b is operated when a fluid is subjected to air supply/water supply from a non-illustrated air supply/water supply channel to assure an observation viewing field of a non-illustrated imaging unit in the distal end hard portion 21. The fluid includes water or a gas.

Furthermore, the grip portion 63 has various kinds of buttons 71 for endoscopic shooting.

The universal cord 65 has a connecting portion 65a connected to a non-illustrated video processor or light source apparatus.

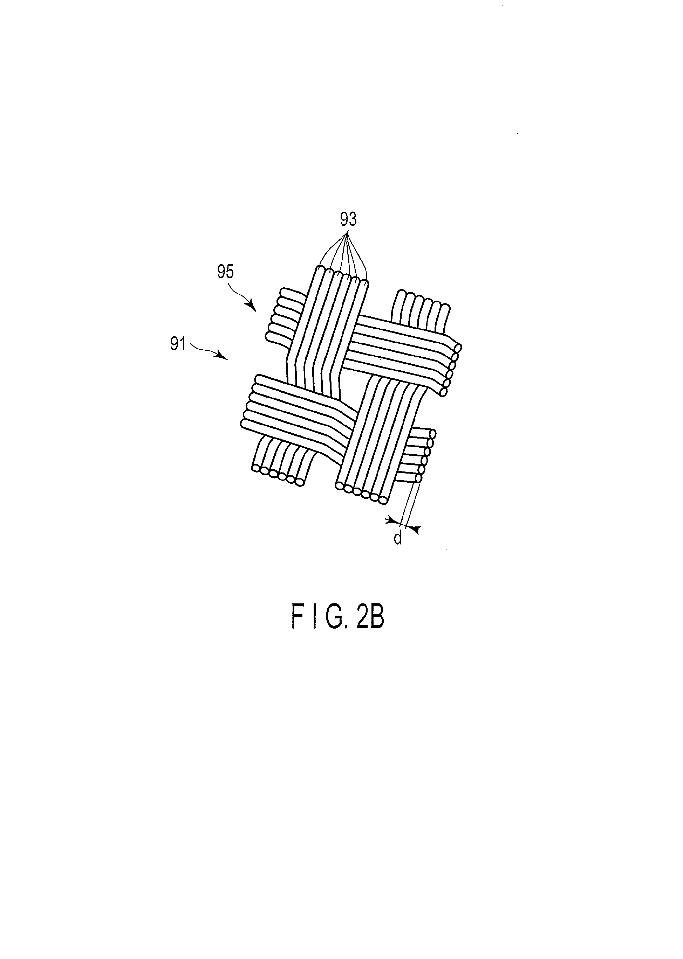

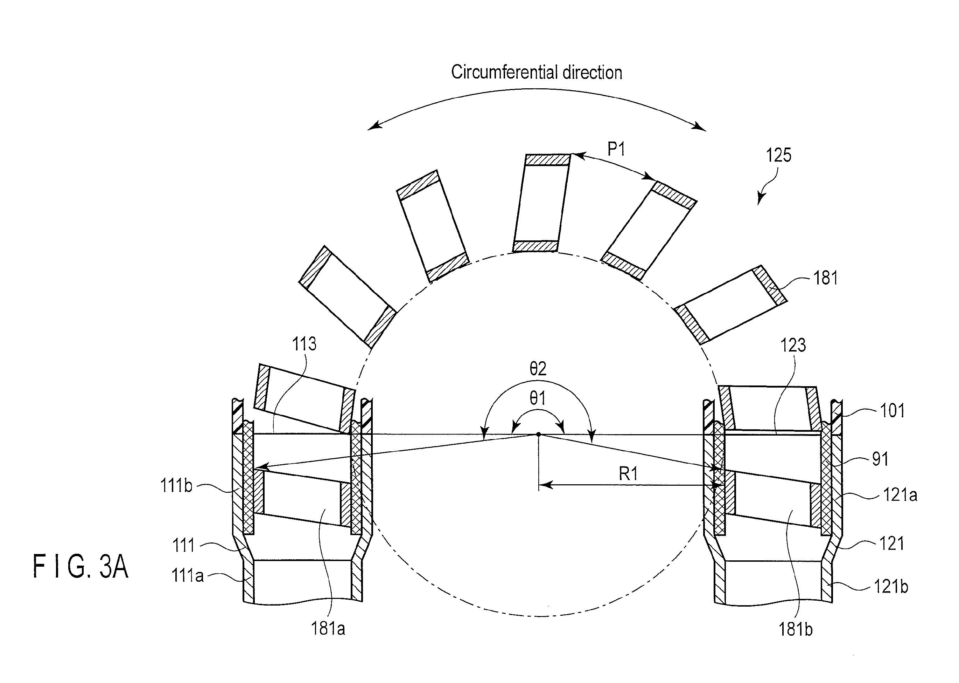

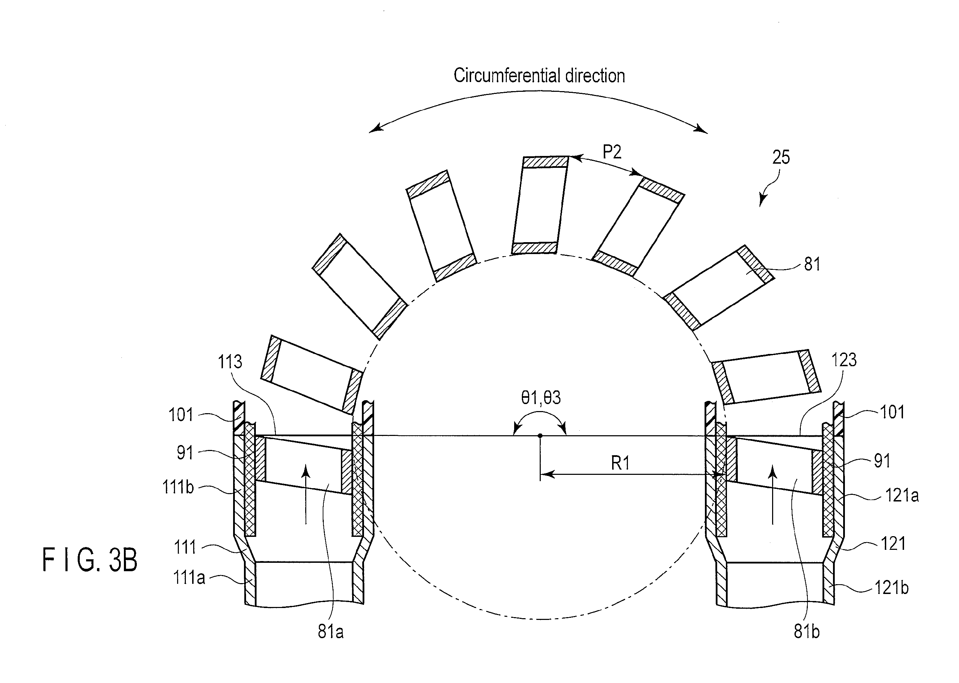

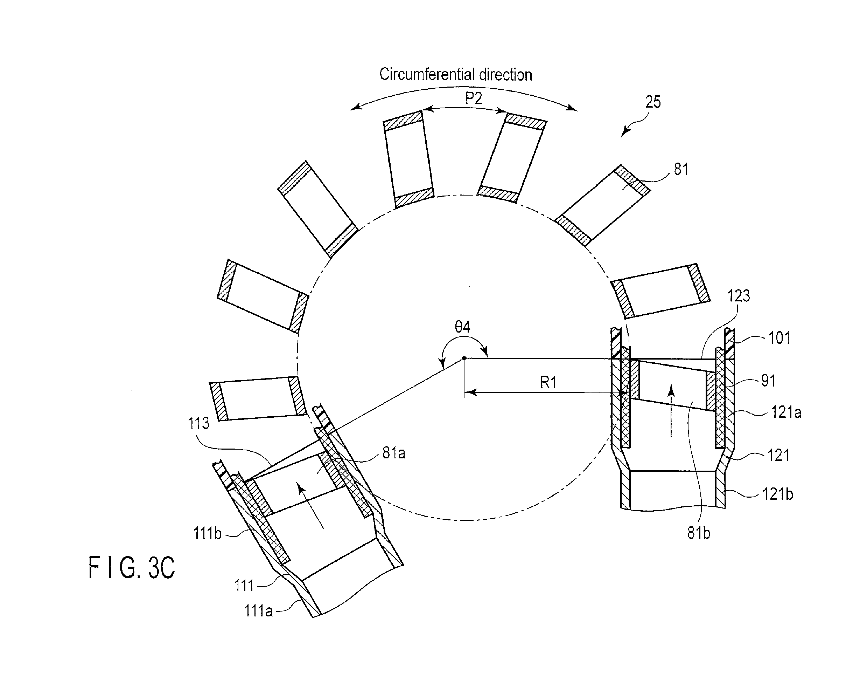

A configuration of the flexible tube portion 25 will now be described with reference to FIG. 2A, FIG. 2B, FIG. 3A, FIG. 3B, and FIG. 3C.

The flexible tube portion 25 has, for example, a hollow shape. In more detail, as shown in FIG. 2A, the flexible tube portion 25 has, for example, a spiral tube 81, a mesh tube 91 which is arranged on the outer side of this spiral tube 81 and laminated on the spiral tube 81, and an envelope 101 which is arranged on the outer side of this mesh tube 91 and laminated on the mesh tube 91. The mesh tube 91 covers the spiral tube 81, and the envelope 101 covers the mesh tube 91.

In this manner, the flexible tube portion 25 has a three-layer configuration formed of the spiral tube 81, the mesh tube 91, and the envelope 101.

The spiral tube 81 is formed into a substantially circular tube shape by molding, for example, a strip-like sheet material into a spiral shape. The sheet material is, for example, a stainless steel material. Each of a distal end portion 81a and a proximal end portion 81b of the spiral tube 81 is cut to form substantially 90 degrees with respect to a central axis of the spiral tube 81. The spiral tube 81 is, for example, a thin-walled metallic spiral tube. The spiral tube 81 is formed into, for example, a loose winding. The spiral tube 81 is arranged in the entire flexible tube portion 25 along a longitudinal (axial) direction of the flexible tube portion 25 to avoid crush of the entire flexible tube portion 25 and local crush of the flexible tube portion 25. The spiral tube 81 has a thickness which is uniform from the distal end portion 81 to the proximal end portion 81b.

As shown in FIG. 2B, the mesh tube 91 is formed by twisting wire bundles 95 into, for example, a substantially circular tube shape. The wire bundle 95 is formed by bundling wires 93. Each wire 93 is made of, for example, a stainless steel material. In the mesh tube 91, the wire bundles 95 are crossed to form a mesh shape. A thickness of this mesh tube 91 corresponds to a value obtained by adding outer diameters of the two wires 93. For example, assuming that d is an outer diameter of the one wire 93, a thickness of the mesh tube 91 is 2d. The mesh tube 91 has a thickness which is uniform from the distal end portion of the mesh tube 91 to a proximal end portion of the mesh tube 91.

The envelope 101 is made of a resin material having flexibility, for example, a rubber material. The envelope 101 is formed into a substantially circular tube shape to cover the outer side of the mesh tube 91.

Moreover, as shown in FIG. 2A and FIG. 3A, the flexible tube portion 25 further has a front connecting portion 111 and a rear connecting portion 121. The front connecting tube 111 is arranged at the distal end portion of the flexible tube portion 25 to couple the proximal end portion of the bend portion 23 with the distal end portion of the flexible tube portion 25, and it is fitted into the node ring 23b arranged on the outermost flexible tube portion 25 side. The rear connecting tube 121 is arranged at the proximal end portion of the flexible tube portion 25 to couple the proximal end portion of the flexible tube portion 25 with the main body portion 61, and it is fixed in the anti-folding portion 61a.

The front connecting tube 111 is arranged on the distal end portion 81a side of the spiral tube 81, and it functions as a first connecting tube into which the distal end portion 81a side is inserted. This front connecting tube 111 is connected to the node ring 25b of the bend portion 23 in the endoscope 1 as described above. It is to be noted that the distal end portion of the mesh tube 91 is also inserted into the front connecting tube 111.

Additionally, the rear connecting tube 121 is arranged on the proximal end portion 81b side of the spiral tube 81, and it functions as a second connecting tube into which the proximal end portion 81b side is inserted. This rear connecting tube 121 is connected to the operating portion 60 in the endoscope 1 as described above. It is to be noted that the proximal end portion of the mesh tube 91 is also inserted into the rear connecting tube 121.

As described above, the front connecting tube 111 and the rear connecting tube 121 are arranged on the distal end portion 81a side and the proximal end portion 81b side of the spiral tube 81, respectively, and they function as a pair of connecting tubes into which both the end portions (the distal end portion 81a and the proximal end portion 81b) of the spiral tube 81 are inserted. The front connecting tube 111 and the rear connecting tube 121 have substantially the same configuration.

As shown in FIG. 2A, an inner diameter of a distal end portion 111a of the front connecting tube 111 is substantially the same as, for example, an outer diameter of the spiral tube 81 and an inner diameter of the mesh tube 91. An outer diameter of the distal end portion 111a of the front connecting tube 111 is substantially the same as, for example, an inner diameter of the node ring 23b. The distal end portion 111a of the front connecting tube 111 is fitted into the node ring 23b. The diameter of the front connecting tube 111 is increased in a stepped pattern from the distal end portion 111a toward a proximal end portion 111b of the front connecting tube 111. An inner diameter of the proximal end portion 111b of the front connecting tube 111 is substantially the same as, for example, an outer diameter of the mesh tube 91 and an inner diameter of the envelope 101. An outer diameter of the proximal end portion 111b of the front connecting tube 111 is substantially the same as, for example, an outer diameter of the node ring 23b and an outer diameter of the envelope 101.

Further, as shown in FIG. 2A, an inner diameter of a distal end portion 121a of the rear connecting tube 121 is substantially the same as, for example, the outer diameter of the mesh tube 91 and the inner diameter of the envelope 101. An outer diameter of the distal end portion 121a of the rear connecting tube 121 is substantially the same as, for example, the outer diameter of the envelope 101. The rear connecting tube 121 is fitted into the anti-folding portion 61a. The diameter of the rear connecting tube 121 is reduced in a stepped pattern from the distal end portion 121a toward a proximal end portion 121b of the rear connecting tube 121. An inner diameter of the proximal end portion 121b of the rear connecting tube 121 is substantially the same as, for example, the outer diameter of the spiral tube 81 and the inner diameter of the mesh tube 91.

Furthermore, as shown in FIG. 2A and FIG. 3B, the front connecting tube 111 has an insertion opening 113 which is arranged at the proximal end portion 111b and into which the distal end portion 81a and the distal end portion of the mesh tube 91 are inserted. Moreover, as shown in FIG. 2A and FIG. 3B, the rear connecting tube 121 has an insertion opening 123 which is arranged at the distal end portion 121a and into which the proximal end portion 81b and the proximal end portion of the mesh tube 91 are inserted.

Moreover, an outer peripheral surface of the distal end portion of the mesh tube 91 is fixed on an inner peripheral surface of the proximal end portion 111b of the front connecting tube 111, for example, bonding. Additionally, an outer peripheral surface of the proximal end portion of the mesh tube 91 is fixed on an inner peripheral surface of the distal end portion 121a of the rear connecting tube 121 by, for example, bonding.

Further, the distal end portion of the envelope 101 is fixed to an edge of the proximal end portion 111b (the insertion opening 113) of the front connecting tube 111 by, for example, bonding. Furthermore, the proximal end portion of the envelope 101 is fixed to an edge of the distal end portion 121a (the insertion opening 123) of the rear connecting tube 121 by, for example, bonding.

It is to be noted that the mesh tube 91 and the envelope 101 are fixed to the front connecting tube 111 and the rear connecting tube 121 by bonding, but the present invention does not have to be restricted to the bonding as long as these tube can be fixed. Therefore, a fixing method is not restricted in particular.

Moreover, the spiral tube 81 is not fixed to the front connecting tube 111 and the rear connecting tube 121, and it is movable with respect to the front connecting tube 111 and the rear connecting tube 121 along the axial direction of the spiral tube 81. It is to be noted that the axial direction of the spiral tube 81 means the longitudinal direction of the flexible tube portion 25, an axial direction of the front connecting tube 111, and an axial direction of the rear connecting tube 121.

As shown in FIG. 3B, when the flexible tube portion 25 bends, the distal end portion 81a moves with respect to the front connecting tube 111 in the axial direction of the spiral tube 81, and the proximal end portion 81b moves with respect to the rear connecting tube 121 in the axial direction of the spiral tube 81. In more detail, the distal end portion 81a slides in the mesh tube 91 fixed to the proximal end portion 111b of the front connecting tube 111. Additionally, the proximal end portion 81b slides in the mesh tube 91 fixed to the distal end portion 121a of the rear connecting tube 121. In this manner, the distal end portion 81a and the proximal end portion 81b slide in the mesh tube 91 fixed to the front connecting tube 111 and the rear connecting tube 121.

Further, in this embodiment, as shown in FIG. 3B, when the flexible tube portion 25 bends, for example, the distal end portion 81a slides in the mesh tube 91 fixed to the proximal end portion 111b of the front connecting tube 111 toward the proximal end portion 81b (the rear connecting tube 121, the grip portion 63). Furthermore, as shown in FIG. 3B, when the flexible tube portion 25 bends, the proximal end portion 81b slides in the mesh tube 91 fixed to the distal end portion 121a of the rear connecting tube 121 toward the distal end portion 81a (the front connecting tube 111, the bend portion 23). Moreover, as shown in FIG. 3B, when the flexible tube portion 25 returns to a linear state shown in FIG. 2A from a bent state shown in FIG. 3B, the distal end portion 81a slides in the mesh tube 91 fixed to the proximal end portion 111b of the front connecting tube 111 toward the bend portion 23, and the proximal end portion 81b slides in the mesh tube 91 fixed to the distal end portion 121a of the rear connecting tube 121 toward the grip portion 63.

It is to be noted that, as shown in FIG. 2A, a pitch (a gap) between sheets of the spiral tube 81 in the axial direction of the spiral tube 81 at the time of, for example, a natural length (the linear state) is L1.

Further, the circumferential direction is not a periaxial direction of the flexible tube portion 25, but it means a bending direction of the flexible tube portion 25 as shown in FIG. 3A and FIG. 3B, namely, a direction along an arc formed by the entire bent flexible tube 25.

Furthermore, as shown in FIG. 3A, a spiral tube that a distal end portion 181a of a spiral tube 181 is fixed to the front connecting tube 111 and a proximal end portion 181b of the spiral tube 181 is fixed to the rear connecting tube 121 will be referred to as a spiral tube 181. Moreover, a flexible tube portion having the spiral tube 181 will be referred to as a flexible tube portion 125.

At this time, the distal end portion 181a of the spiral tube 181 is fixed to, for example, the distal end portion 111a side rather than the insertion opening 113 so that it cannot come off the front connecting tube 111. Additionally, the proximal end portion 181b of the spiral tube 181 is fixed to, for example, the proximal end portion 121b side rather than the insertion opening 123 so that it cannot come off the rear connecting tube 121.

As shown in FIG. 3A, it is assumed that the flexible tube portion 125 bends with a radius of curvature R1 and a bending angle θ1. It is assumed that bending angle θ1 is, for example, 180 degrees. This bending angle θ1 represents, for example, an angle formed between a flat surface where the insertion opening 113 is formed and a flat surface where the insertion opening 123 is arranged. Additionally, the radius of curvature R1 represents, for example, a distance from the center of the bent flexible tube portion 125 to an outer peripheral surface of the spiral tube 181. In a circumferential direction of the spiral tube 181, a pitch (a gap) between the sheets on the outer peripheral side is assumed to be P1. This P1 generally increases beyond L1.

Further, at this time, as described above, the distal end portion 181a of the spiral tube 181 is fixed to the front connecting tube 111, and the proximal end portion 181b of the spiral tube 181 is fixed to the rear connecting tube 121. Therefore, a bending angle θ2 of the spiral tube 181 is larger than bending angle θ1. Bending angle θ2 represents an angle formed between the distal end portion 181a and the proximal end portion 181b in the spiral tube 181, for example.

Furthermore, as shown in FIG. 3B, when the flexible tube portion 25 like this embodiment bends with the same radius of curvature R1 at the same bending angle θ1 as the flexible tube portion 125, a pitch (a gap) between sheets on the outer peripheral side is P2 in the circumferential direction of the spiral tube 81. This P2 increases beyond L1. However, in the spiral tube 81 according to this embodiment, the distal end portion 81a slides in the mesh tube 91 fixed to the proximal end portion 111b of the front connecting tube 111 toward the proximal end portion 81b (the insertion opening 113, the rear connecting tube 121, the grip portion 63), and the proximal end portion 81b slides in the mesh tube 91 fixed to the distal end portion 121a of the rear connecting tube 121 toward the distal end portion 81a (the insertion opening 123, the front connecting tube 111, the bend portion 23). Therefore, in the circumferential direction, the number of turns (sheets) of the spiral tube 81 per circumference increases beyond the number of turns (sheets) of the spiral tube 181. As a result, in the spiral tube 81 according to this embodiment, the pitch (the gap) between the sheets is narrower than that of the spiral tube 181, and the sheets are closely arranged in the circumferential direction as compared with the spiral tube 181. Therefore, P2 is smaller (narrower) than P1.

In this manner, P1>P2>L1 is achieved.

Further, for example, as shown in FIG. 3B, the distal end portion 81a slides in the mesh tube 91 fixed to the proximal end portion 111b of the front connecting tube 111 toward the proximal end portion 81b and the proximal end portion 81b slides in the mesh tube 91 fixed to the distal end portion 121a of the rear connecting tube 121 toward the distal end portion 81a as described above. Therefore, a bending angle θ3 of the spiral tube 81 can be substantially the same as bending angle θ1. Furthermore, bending angle θ3 can be smaller than bending angle 82. Bending angle θ3 represents an angle formed between the distal end portion 81a and the proximal end portion 81b of the spiral tube 81, for example.

As described above, θ2>θ3≥θ1 is achieved.

Further, as shown in FIG. 3C, assuming that both the radius of curvature of the flexible tube portion 25 and the radius of curvature of the flexible tube portion 125 are R1, P1=P2, a bending angle θ4 (for example, 210 degrees) of the flexible tube portion 25 is larger than bending angle θ1 (for example, 180 degrees) of the flexible tube portion 125. That is, the flexible tube portion 25 bends at a larger angle than the flexible tube portion 125.

Furthermore, in cases where the flexible tube portion 25 bends as shown in FIG. 3B and where the flexible tube portion 25 returns to such a linear state as shown in FIG. 2A from such a bent state as shown in FIG. 3B, L2 is longer than L3 as shown in FIG. 2A so that the distal end portion 81a and the proximal end portion 81b can slide, the distal end portion 81a can be prevented from coming off the front connecting tube 111, the proximal end portion 81b can be prevented from coming off the rear connecting tube 121, and the spiral tube 81 can be arranged in the entire flexible tube portion 25. L2 represents a length of the entire spiral tube 81 from the distal end portion 81a to the proximal end portion 81b in the straight spiral tube 81. Furthermore, L3 represents a length from the insertion opening 113 which is formed at the proximal end portion 111b of the front connecting tube 111 and into which the distal end portion 81a side is inserted to the insertion opening 123 which is formed at the distal end portion 121a of the rear connecting tube 121 and in which the proximal end portion 81b side is inserted in the straight spiral tube 81. Each of L2 and L3 represents a length along the axial direction of the spiral tube 81.

It is to be noted that, as shown in FIG. 3B, in cases where the flexible tube portion 25 bends and the distal end portion 81a slides in the mesh tube 91 fixed at the proximal end portion 111b of the front connecting tube 111 toward the proximal end portion 81b, the front connecting tube 111 prevents the distal end portion 81a from coming off the front connecting tube 111 (the insertion opening 113). Therefore, the front connecting tube 111 has a length that prevents the sliding distal end portion 81a from coming off the front connecting tube 111 or has a preventing portion such as a stopper that prevents the distal end portion 81a from coming off the front connecting portion 111.

Moreover, as shown in FIG. 3B, in cases where the flexible tube portion 25 bends and the proximal end portion 81b slides in the mesh tube 91 fixed at the distal end portion 121a of the rear connecting tube 121 toward the distal end portion 81a, the rear connecting tube 121 prevents the proximal end portion 81b from coming off the rear connecting tube 121 (the insertion opening 123). Therefore, the rear connecting tube 121 has a length that prevents the sliding proximal end portion 81b from coming off the rear connecting tube 121 or has a preventing portion such as a stopper that prevents the proximal end portion 81b from coming off the rear connecting tube 121.

Additionally, in cases where the flexible tube portion 25 returns to the linear state shown in FIG. 2A from the bent state shown in FIG. 3B and the distal end portion 81a slides in the mesh tube 91 fixed at the proximal end portion 111b of the front connecting tube 111 toward the bend portion 23, the distal end portion 81a may be inserted into the node ring 23b through the front connecting tube 111. Alternatively, the front connecting tube 111 may prevent the distal end portion 81a from coming off the front connecting tube 111 (the distal end portion 111a) toward the bend portion 23. At this time, the front connecting tube 111 has a preventing portion such as a stopper that prevents the sliding distal end portion 81a from being inserted into the node ring 23b through the front connecting tube 111. Alternatively, the front connecting tube 111 is formed in such a manner that an inner diameter of the distal end portion 111a of the front connecting tube 111 becomes narrower than an outer diameter of the distal end portion 81a of the spiral tube 81.

Additionally, in cases where the flexible tube portion 25 returns to the linear state shown in FIG. 2A from the bent state shown in FIG. 3B and the proximal end portion 81b slides in the mesh tube 91 fixed at the distal end portion 121a of the rear connecting tube 121 toward the grip portion 63, the proximal end portion 81b may be inserted into the grip portion 63 side through the rear connecting tube 121. Alternatively, the rear connecting tube 121 may prevent the proximal end portion 81b from coming off the rear connecting tube 121 (the proximal end portion 121b) toward the grip portion 63 side. At this time, the rear connecting tube 121 has a preventing portion such as a stopper that prevents the sliding proximal end portion 81b from being inserted to the grip portion 63 side through the rear connecting tube 121. Alternatively, the rear connecting tube 121 is formed in such a manner that an inner diameter of the proximal end portion 121b of the rear connecting tube 121 becomes narrower than an outer diameter of the proximal end portion 81b of the spiral tube 81.

The preventing portion in each of the front connecting tube 111 and the rear connecting tube 121 is, for example, a non-illustrated protruding portion formed on each of the inner peripheral surface of the front connecting tube 111 and the inner peripheral surface of the rear connecting tube 121. The protruding portion is integral with respect to, for example, each of the front connecting tube 111 and the rear connecting tube 121.

How the spiral tube 81 in this embodiment bends will now be described with reference to FIG. 2A, FIG. 3A, FIG. 3B, and FIG. 3C.

As shown in FIG. 3B, the flexible tube portion 25 bends in such a manner that both the radius of curvature of the flexible tube portion 25 and the radius of curvature of the flexible tube portion 125 become R1 and both the bending angle of the flexible tube portion 25 and the bending angle of the flexible tube portion 125 become θ1. At this time, the spiral tube 81 also bends and, at the same time, the distal end portion 81a slides in the mesh tube 91 fixed at the proximal end portion 111b of the front connecting tube 111 toward the proximal end portion 81b (the rear connecting tube 121, the grip portion 63), and the proximal end portion 81b slides in the mesh tube 91 fixed at the distal end portion 121a of the rear connecting tube 121 toward the distal end portion 81a (the front connecting tube 111, the bend portion 23).

As a result, in the circumferential direction, the number of turns (sheets) of the spiral tube 81 per circumference increases beyond the number of turns (sheets) of the spiral tube 181. Therefore, the spiral tube 81 is more densely arranged along the circumferential direction than the spiral tube 181. Additionally, P2 is smaller (narrower) than P1. Further, bending angle θ3 of the spiral tube 81 can be substantially the same as bending angle θ1. Furthermore, bending angle θ3 can become smaller than bending angle θ2.

When P1>P2 and θ2>θ3>θ1 are achieved, the flexible tube portion 25 (the spiral tube 81) can more flexibly bend than the flexible tube portion 125 (the spiral tube 181) and can more easily bend than the flexible tube portion 125 (the spiral tube 181). Therefore, force that bends the flexible tube portion 25 (the spiral tube 81) is smaller than force that bends the flexible tube portion 125 (the spiral tube 181), thus improving the operability.

Furthermore, at the time of inserting the flexible tube portion 25 into, for example, the sigmoid colon or the like while bending, the flexible tube portion 25 flexibly bends in accordance with the sigmoid colon as described above. Therefore, the burden on the patient at the time of using the flexible tube portion 25 becomes smaller than that at the time of using the flexible tube portion 125. In this manner, the burden on the patient becomes small.

It is to be noted that, assuming that P1=P2, as shown in FIG. 3C, the spiral tube 81 is able to bend more than the spiral tube 181. Therefore, the flexible tube portion 25 bends in accordance with the sigmoid colon, thereby improving the operability.

Moreover, since L2>L3 is achieved, the distal end portion 81a and the proximal end portion 81b assuredly slide, the distal end portion 81a is prevented from coming off the front connecting tube 111, and the proximal end portion 81b is prevented from coming off the rear connecting tube 121. Additionally, since L2>L3 is achieved, even if the flexible tube portion 25 bends, the spiral tube 81 is arranged in the entire flexible tube portion 25, thereby avoiding crush of the entire flexible tube portion 25 and local crush of the flexible tube portion 25.

Further, when the spiral tube 81 is loosely wound, the spiral tube 81 can more flexibly bend.

Furthermore, when the flexible tube portion 25 bends as shown in FIG. 3B, the distal end portion 81a is prevented from coming off the front connecting tube 111 (the insertion opening 113) toward the proximal end portion 81b by the front connecting tube 111. Moreover, as shown in FIG. 3B, when the flexible tube portion 25 bends, the proximal end portion 81b is prevented from coming off the rear connecting tube 121 (the insertion opening 123) toward the distal end portion 81a by the rear connecting tube 121.

Additionally, when the flexible tube portion 25 returns to such a linear state as shown in FIG. 2A from such a bent state as shown in FIG. 3B, the distal end portion 81a is inserted into the node ring 23b through the front connecting tube 111. Alternatively, the distal end portion 81a is prevented from coming off the front connecting tube 111 (the distal end portion 111a) toward the bend portion 23 by the front connecting tube 111. Further, when the flexible tube portion 25 returns to such a linear state as shown in FIG. 2A from such a bent state as shown in FIG. 3B, the proximal end portion 81b is inserted into the grip portion 63 side through the rear connecting tube 121, or the proximal end portion 81b is prevented from coming off the rear connecting tube 121 (the proximal end portion 121b) toward the grip portion 63 side by the rear connecting tube 121.

As a result, when the flexible tube portion 25 again bends, the distal end portion 81a assuredly slides in the mesh tube 91 fixed at the proximal end portion 111b of the front connecting tube 111, and the proximal end portion 81b assuredly slides in the mesh tube 91 fixed at the distal end portion 121a of the rear connecting tube 121. Therefore, the improvement in operability and the reduction in burden on the patient can be constantly maintained.

As described above, in this embodiment, when the flexible tube portion 25 bends, along the axial direction of the spiral tube 81, the distal end portion 81a which is inserted into the front connecting tube 111 slides in the mesh tube 91 fixed at the proximal end portion 111b of the front connecting tube 111, and the proximal end portion 81b which is inserted into the rear connecting tube 121 slides in the mesh tube 91 fixed at the distal end portion 121a of the rear connecting tube 121. As a result, in this embodiment, when the flexible tube portion 25 bends, along the circumferential direction of the spiral tube 81, the number of turns (sheets) of the spiral tube 81 per circumference can increase beyond the number of turns (sheets) of the spiral tube 181, the spiral tube 81 can be thereby more densely arranged along the circumferential direction than the spiral tube 181, and P2 can be reduced to be smaller (narrower) than P1. Furthermore, in this embodiment, θ2>θ3≥θ1 can be achieved. Therefore, in this embodiment, the flexible tube portion 25 can more flexibly bend than the flexible tube portion 125, and the flexible tube portion 25 can more flexibly bend than the flexible tube portion 125. Therefore, in this embodiment, force that bends the flexible tube portion 25 can be reduced to be smaller than force that bends the flexible tube portion 125, and large force that bends the flexible tube portion 25 is not required at the time of bending the flexible tube portion 25 with a small radius of curvature, and the operability can be improved even in case of inserting the flexible tube portion 25 into the sigmoid colon. Moreover, as a result, in this embodiment, even in case of inserting the flexible tube portion 25 into the sigmoid colon, the burden on the patient can be reduced.

Additionally, in this embodiment, as shown in FIG. 3C, when the radius of curvature of the spiral tube 81 and the radius of curvature of the spiral tube 181 are R1, P1=P2, the spiral tube 81 is able to bend more largely bend than the spiral tube 181. As a result, in this embodiment, even in case of inserting the flexible tube portion 25 into the sigmoid colon, as described above, the operability can be improved, and the burden on the patient can be reduced.

Further, in this embodiment, the distal end portion 81a which is inserted into the front connecting tube 111 slides in the mesh tube 91 fixed at the proximal end portion 111b of the front connecting tube 111, and the proximal end portion 81b which is inserted into the rear connecting tube 121 slides in the mesh tube 91 fixed at the distal end portion 121a of the rear connecting tube 121. Therefore, in this embodiment, bending angle θ4 of the spiral tube 81 can be freely changed in accordance with the sigmoid colon. At the same time, in this embodiment, the flexible tube portion 25 can have more latitude (flexibility) in bending than the flexible tube portion 125. Therefore, in this embodiment, the flexible tube portion 25 can bend in accordance with the sigmoid colon.

Furthermore, in this embodiment, when L2>L3 is set, in cases where the flexible tube portion 25 bends as shown in FIG. 3B and where the flexible tube portion 25 returns to such a linear state as shown in FIG. 2A from such a bent state as shown in FIG. 3B, the distal end portion 81a can slide in the mesh tube 91 fixed at the proximal end portion 111b of the front connecting tube 111, and the proximal end portion 81b can slide in the mesh tube 91 fixed at the distal end portion 121a of the rear connecting tube 121. Moreover, in this embodiment, when L2>L3 is set, the distal end portion 81a can be prevented from coming off the front connecting tube 111, and the proximal end portion 81b can be prevented from coming off the rear connecting tube 121. Additionally, in this embodiment, when L2>L3 is set, the spiral tube 81 can be arranged over the entire flexible tube 25, and crush of the entire flexible tube portion 25 and local crush of the flexible tube portion 25 can be avoided.

Additionally, in this embodiment, when the spiral tube 81 is formed as a loose winding, the spiral tube 81 can more flexibly bend.

Further, in this embodiment, as shown in FIG. 3B, when the flexible tube portion 25 bends, the distal end portion 81a can be prevented from coming off the insertion opening 113 by the front connecting tube 111, and the proximal end portion 81b can be prevented from coming off the insertion opening 123 by the rear connecting tube 121. Furthermore, in this embodiment, when the flexible tube portion 25 returns to such a linear state as shown in FIG. 2A from such a bent state as shown in FIG. 3B, the distal end portion 81a can be inserted into the node ring 23b through the front connecting tube 111, and the proximal end portion 81b can be inserted into the grip portion 63 side through the rear connecting tube 121. Moreover, in this embodiment, the front connecting tube 111 can prevent the distal end portion 81a from coming off the distal end portion 111a toward the bending portion 23, and the rear connecting tube 121 can prevent the proximal end portion 81b from coming off the proximal end portion 121b toward the grip portion 63 side. Therefore, in this embodiment, when the flexible tube portion 25 again bends, the distal end portion 81a can assuredly slide in the mesh tube 91 fixed at the proximal end portion 111b of the front connecting tube 111, and the proximal end portion 81b can assuredly slide in the mesh tube 91 fixed at the distal end portion 121a of the rear connecting tube 121. Therefore, in this embodiment, the improvement in operability and the reduction in burden on the patient can be constantly maintained.

Additionally, in this embodiment, since the distal end portion 81a slides in the mesh tube 91 fixed at the proximal end portion 111b of the front connecting tube 111, the front connecting tube 111 can be prevented from becoming worn away by the sliding distal end portion 81a when the distal end portion 81a slides. Therefore, in this embodiment, damage to the spiral tube 81 can be avoided. This point can be likewise applied to the proximal end portion 81b, the mesh tube 91 fixed at the proximal end portion 121b of the rear connecting tube 121, and the rear connecting tube 121.

It is to be noted that both the distal end portion 81a and the proximal end portion 81b slide in this embodiment, but the present invention is not restricted thereto. In this embodiment, for example, the distal end portion 81a may slide in the mesh tube 91 fixed at the proximal end portion 111b of the front connecting tube 111, and the proximal end portion 81b may be fixed to the rear connecting tube 121. This fixation is realized by, for example, welding or bonding.

As a result, in this embodiment, at the distal end portion of the flexible tube portion 25 which is the first portion in the flexible tube portion 25 that is inserted into a body cavity, the flexible tube portion 25 can be more flexibly bent from the distal end portion side.

It is needless to say that, in this embodiment, for example, the distal end portion 81a may be fixed to the front connecting tube 111 and the proximal end portion 81b alone may slide in the mesh tube 91 fixed at the distal end portion 121a of the rear connecting tube 121. This fixation is realized by, for example, welding or bonding.

For example, when the distal end portion 81a is inserted into the plurality of node rings 23a and 23b arranged on the proximal end portion side of the bend portion 23, bending resistance is increased on the proximal end portion side of the bend portion 23, and the proximal end portion side of the bend portion 23 may possibly have a problem in bending. Furthermore, when a length of a coupling portion (the front connecting tube 111) of the flexible tube portion 25 and the bend portion 23 becomes sufficiently long so that the distal end portion 81a can slide, the hard portion which does not bend becomes long. However, in this embodiment, when the distal end portion 81a is fixed to the front connecting tube 111, the proximal end portion side of the bend portion 23 can be prevented from having a problem in bending, and an increase in length of the hard portion can be avoided.

Moreover, in this embodiment, when the distal end portion 81a is fixed to the front connecting tube 111, the distal end portion 81a can be assuredly prevented from being inserted into the node rings 23a and 23b, and the distal end portion 81a inserted in the node rings 23a and 23b can be prevented from posing a problem for bending of the bend portion 23.

Additionally, in this embodiment, although a sheet corresponding to one turn alone is inserted into the front connecting tube 111 to slide on the distal end portion 81a side, this number of turns (helixes, sheets) is not restricted as long as the distal end portion 81a side is inserted, can slide, and does not come off the front connecting tube 111. This point is likewise applied to the proximal end portion 81b side.

As described above, in this embodiment, the distal end portion 81a side slides in the mesh tube 91 fixed at the proximal end portion 111b of the front connecting tube 111 along the axial direction of the spiral tube 81, and/or the proximal end portion 81b side slides in the mesh tube 91 fixed at the distal end portion 121a of the rear connecting tube 121 along the axial direction of the spiral tube 81.

It is to be noted that this embodiment is not restricted to the above example as long as the distal end portion 81a moves with respect to the front connecting tube 111 in the axial direction of the spiral tube 81, and/or the proximal end portion 81b moves with respect to the rear connecting tube 121 in the axial direction of the spiral tube 81 when the flexible tube portion 25 bends.

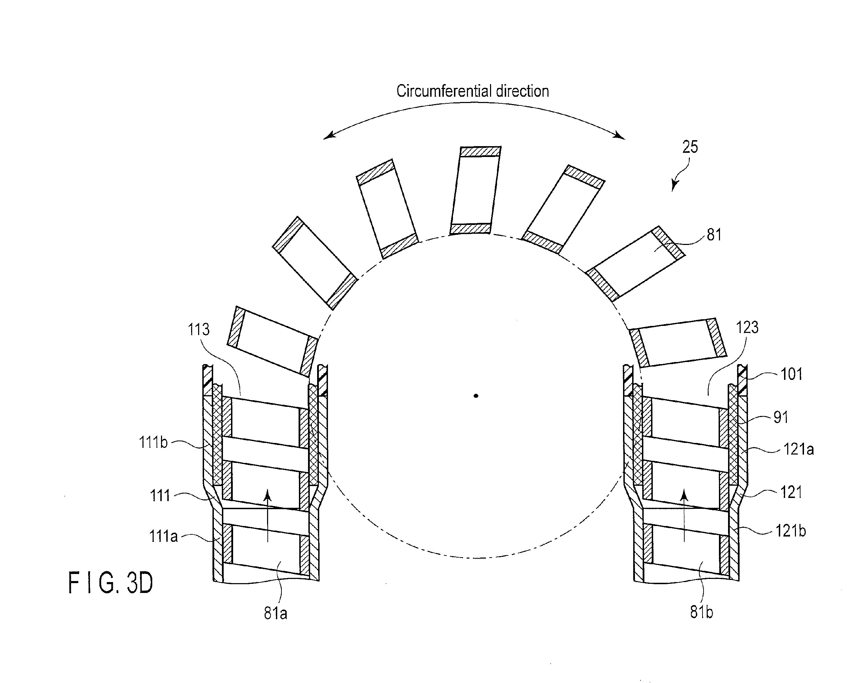

For example, as shown in FIG. 3D, the distal end portion 81a is arranged to reach the distal end portion 111a, and the proximal end portion 81a is arranged to reach the proximal end portion 121b. Further, when the flexible tube portion 25 bends, the distal end portion 81a side may directly slide in the distal end portion 111a of the front connecting tube 111 along the axial direction of the spiral tube 81, and/or the proximal end portion 81b side may directly slide in the proximal end portion 121b of the rear connecting tube 121 along the axial direction of the spiral tube 81.

It is to be noted that, if the distal end portion 81a side can slide in the front connecting tube 111 along the axial direction of the spiral tube 81 when the flexible tube portion 25 bends, a slide position is not restricted in particular. This point is likewise applied to the proximal end portion 81 side.

As described above, the spiral tube 81 can move in the axial direction of the spiral tube 81 with respect to at least one of the front connecting tube 111 and the rear connecting tube 121.

Furthermore, in this embodiment, although the spiral tube 81 is formed as a loose winding, the present invention is not restricted thereto. The spiral tube 81 may be formed as, for example, a compact winding, or the spiral tube 81 may be formed of, for example, a close coil having initial tension provided thereto. Even in such a case, this embodiment can obtain the above-described effect. It is to be noted that, when the spiral tube 81 is formed of a close coil, the close coil is, for example, a close coil spring. The close coil is a spiral wire rod formed of a spiral wire 93.

Furthermore, in this embodiment, the shape of the front connecting tube 111 and the shape of the rear connecting tube 121 are not restricted in particular as long as the mesh tube 91 and the envelope 101 can be fixed and the spiral tube 81 can slide as described above. Moreover, in this embodiment, if the spiral tube 81 can slide as described above, fixing positions of the mesh tube 91 and the envelope 101 on the front connecting tube 111 and the rear connecting tube 121 are not restricted in particular.

Moreover, in this embodiment, although the front connecting tube 111 is fitted in the node ring 23b arranged on the outermost flexible tube portion 25 side, coupling is not restricted in particular as long as the front connecting tube 111 and the node ring 23b can be coupled.

Additionally, in this embodiment, although the distal end portion 81a is inserted into the front connecting tube 111 and the proximal end portion 81b is inserted into the rear connecting tube 121, the present invention is not restricted thereto. For example, the distal end portion 81a may be inserted into the node ring 23b arranged on the outermost flexible tube portion 25 side, and the proximal end portion 81b may be inserted into the insertion opening in the anti-folding portion 61a. In this case, the node ring 23b functions as the front connecting tube 111, and the insertion opening 113 functions as the rear connecting tube 121.

Further, in this embodiment, the endoscope 1 is used for the medical purpose. Therefore, in the flexible tube portion 25, the mesh tube 91 covers the spiral tube 81, and the envelope 101 covers the mesh tube 91. However, the configuration of the flexible tube portion 25 is not restricted in particular as long as the mesh tube 91 and the envelope 101 cover the spiral tube 81. For example, in the light of using the endoscope 1 for the industrial purpose, the envelope 101 as a resin layer may cover the spiral tube 81, and the mesh tube 91 may cover the envelope 101.

In this case, no problem may arise as long as both end portions of at least one of the mesh tube 91 and the envelope 101 which is a resin layer are fixed to the front connecting tube 111 and the rear connecting tube 121.

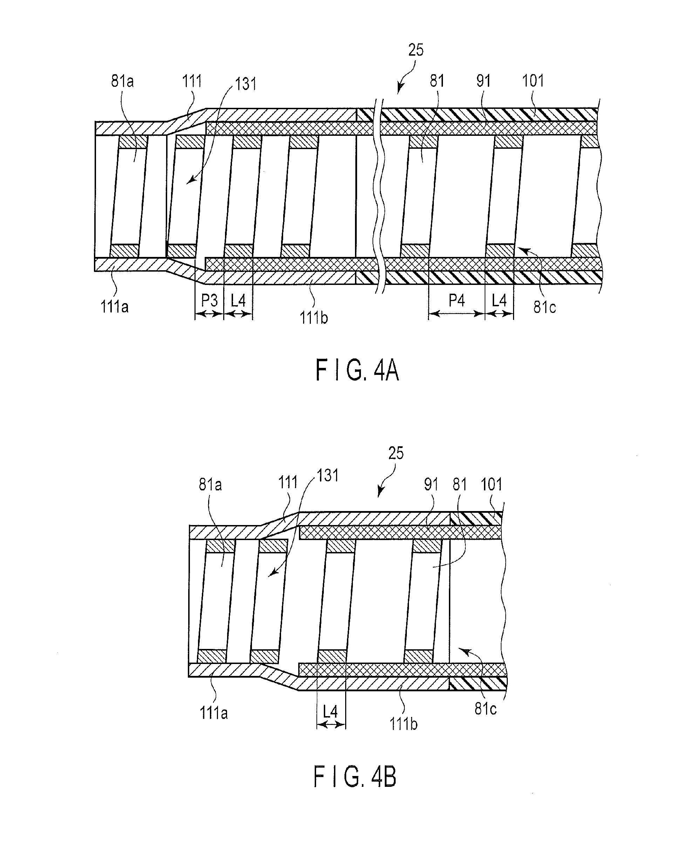

A second embodiment according to the present invention will now be described with reference to FIG. 4A.

As shown in FIG. 4A, for example, a distal end portion 81a side is fixed to a front connecting tube 111, and it is formed as an elastic portion 131 which has a lower spring constant than that of the spiral tube 81. The spring constant of the spiral tube 81 represents, for example, a spring constant in an intermediate portion 81c of the spiral tube 81 placed between the distal end portion 81a and a proximal end portion 81b. It is to be noted that, because of a relationship of the spring constant, the elastic portion 131 (the distal end portion 81a side) greatly expands or contracts as compared with an intermediate portion 81c when the flexible tube portion 25 bends or the flexible tube portion 25 returns to a linear state from a bent state.

The distal end portion 81a side is inserted into the front connecting tube 111. Moreover, part of the distal end portion 81a side is fixed to, for example, the distal end portion 111a of the front connecting tube 11 by bonding or welding. Additionally, the other portion of the distal end portion 81a side moves (slides) like the first embodiment. The distal end portion 81a side is integral with the spiral tube 81. Since the spring constant on the distal end portion 81a side is lower than the spring constant of the spiral tube 81, the distal end portion 81a side is formed into, for example, a compact winding as compared with the intermediate portion 81c.

Therefore, for example, as shown in FIG. 4A, in an axial direction of the spiral tube 81, when lengths of sheets of the entire spiral tube 81 are L4 and uniform, a pitch P3 between the sheets on the distal end portion 81a side is narrower than a pitch P4 between the sheets in the intermediate portion 81c. As described above, the spring constant of the distal end portion 81a is lower than the spring constant of the intermediate portion 81c.

In this embodiment, when the distal end portion 81a side is formed as the elastic portion 131 having the spring constant lower than that of the spiral tube 81, flexibility of the flexible tube portion 25 can be varied (adjusted) at the time of bending the flexible tube portion 25.

It is to be noted that, in this embodiment, part of the distal end portion 81a alone is fixed at a distal end portion of a fist connecting tube, other portions of the distal end portion 81a can slide like the first embodiment.

It is to be noted that this embodiments does not have to be restricted to the above example. As a first modification, when lengths of sheets on the entire spiral tube 81 are uniform as indicated by L4 as shown in FIG. 4B, a pitch between the sheets on the distal end portion 81a side may be gradually lowered from the intermediate portion 81c side toward the bend portion 23 (the distal end portion 81a) side. That is, on the distal end portion 81a side of the spiral tube 81, the spring constant is gradually lowered from the intermediate 81c side toward the bend portion 23 (the distal end portion 81a) side.

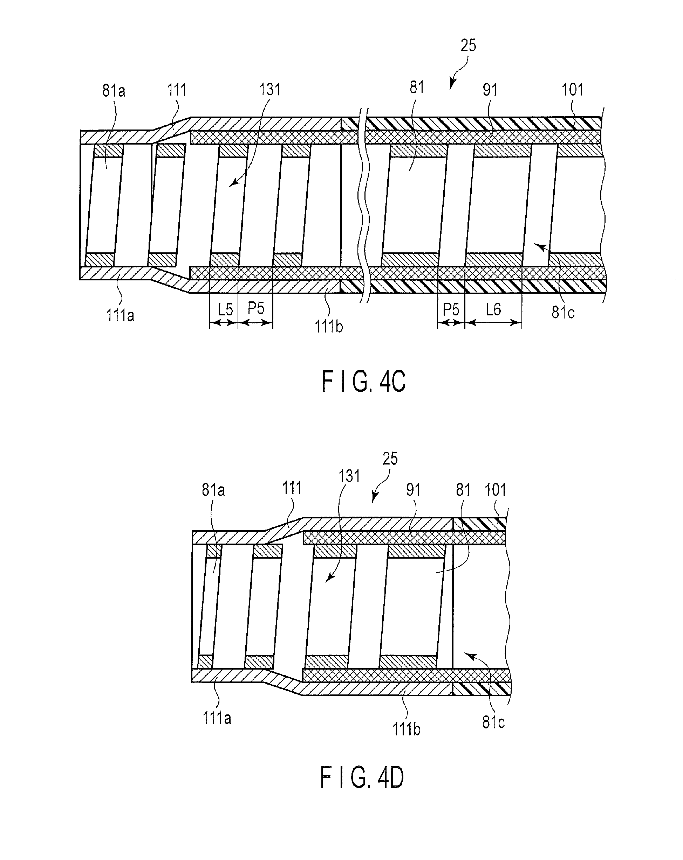

Further, as a second modification, for example, when a pitch between the sheets of the entire spiral tube 81 is uniform as indicated by P5 in the axial direction of the spiral tube 81 as shown in FIG. 4C, a length L5 of the sheet on the distal end portion 81a side may be shorter than a length L6 of the sheet in the intermediate portion 81c. As described above, the spring constant of the distal end portion 81a is lower than the spring constant of the intermediate portion 81c.

Moreover, as a third modification, for example, when a pitch between the sheets of the entire spiral tube 81 is uniform as shown in FIG. 4D, a length of the sheet on the distal end portion 81a side may be gradually reduced from the intermediate portion 81c side toward the bend portion 23 (the distal end portion 81a) side as shown in FIG. 4D. That is, on the distal end portion 81a side of the spiral tube 81, the spring constant is gradually reduced from the intermediate portion 81c side toward the bend portion 23 (the distal end portion 81a) side.

It is to be noted that the description has been given as to the example of the distal end portion 81a side in this embodiment and the first to third modifications, but the present invention is not restricted thereto, and the proximal end portion 81b side may be formed as the elastic portion 131. That is, in this embodiment and the first to third modifications, when the flexible tube portion 25 bends or when the flexible tube portion 25 returns to the linear state from the bent state, at least one of the distal end portion 81a and the proximal end portion 81b of the spiral tube 81 is fixed to the connecting tube (the front connecting tube 111, the rear connecting tube 121) through the second elastic portion of the spiral tube 81 that stretches or contracts than the first elastic portion of the spiral tube 81. The first elastic portion represents the intermediate portion 81c. Furthermore, the second elastic portion represents the elastic portion 131, i.e., the distal end portion 81a side and the proximal end portion 81b side. In other words, of the distal end portion 81a side and the proximal end portion 81b side, a sliding end portion side can be fixed to the connecting tube in which this end portion is inserted and slides, and it can function as the elastic portion 131 having a spring constant lower than that of the spiral tube 81.

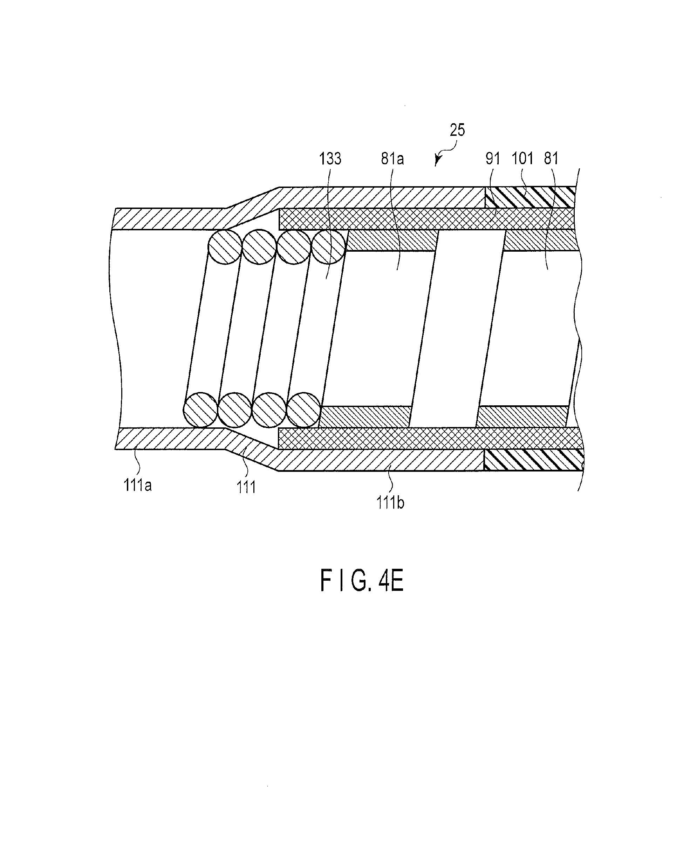

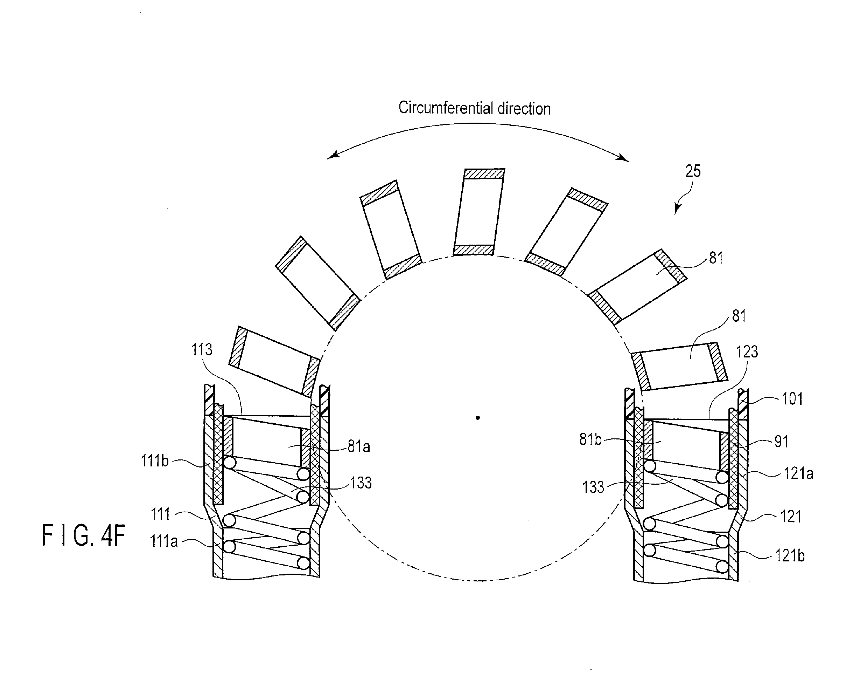

Moreover, as a fourth modification, the flexible tube portion 25 has an elastic member 133 which is connected to the distal end portion 81a, inserted into the front connecting tube 111, fixed to the front connecting tube 111, and has a spring constant lower than that of the spiral tube 81 as shown in FIG. 4E. Because of such a spring constant relation, when the flexible tube portion 25 bends or when the flexible tube portion 25 returns to the linear state from the bent state, the elastic member 133 stretches or contracts more than the spiral tube 81. The elastic member 133 is inserted into the front connecting tube 111 and fixed to, for example, the distal end portion 111a of the front connecting tube 111 by bonding, welding, or the like. The elastic member 133 is different fro the spiral tube 81. The elastic member 133 is, for example, a coil spring as a loose winding. As shown in FIG. 4F, the elastic member 133 stretches along the axial direction of the spiral tube 81 when the flexible tube portion 25 bends. The elastic member 133 contracts along the axial direction of the spiral tube 81 when the flexible tube portion 25 returns to the linear state from the bent state. In this manner, the elastic member 133 stretches or contracts.

As described above, in this modification, the flexibility of the flexible tube portion 25 at the time of bending the flexible tube portion 25 can be changed (adjusted) by using elasticity of the elastic member 133.

It is to be noted that the example of the distal end portion 81a has been described in this modification, but the present invention does have to be restricted thereto, and the same configuration can be used for the proximal end portion 81b. That is, at least one of the distal end portion 81a and the proximal end portion 81b of the spiral tube 81 is fixed to the connecting tube (the front connecting tube 111, the rear connecting tube 121) through the elastic member 133 that stretches or contracts more than the spiral tube 81 when the flexible tube portion 25 bends or when the flexible tube portion 25 returns to the linear state from the bent state. In other words, in this embodiment, the elastic portion 131 is connected to a sliding one of the distal end portion 81a and the proximal end portion 81b, inserted into the connecting tube of the sliding end portion, and fixed to this connecting tube.

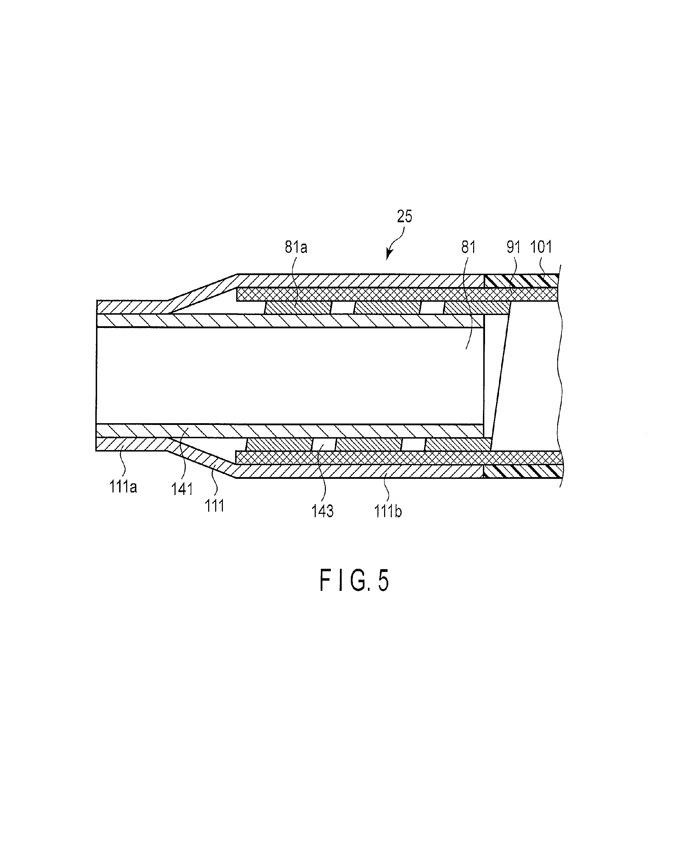

A third embodiment according to the present invention will now be described with reference to FIG. 5.

A flexible tube portion 25 according to this embodiment further has a hollow member 141 which is narrower than a front connecting tube 111, inserted into the front connecting tube 111, and arranged in the front connecting tube 111. An axial direction of the hollow member 141 is arranged along an axial direction of a spiral tube 81. The hollow member 141 is, for example, a cylindrical member having a uniform thickness. The hollow member 141 is, for example, a metallic pipe. An outer diameter of the hollow member 141 is substantially the same as, for example, an inner diameter of a distal end portion 111a of the front connecting tube 111, and the hollow member 141 is fitted in the distal end portion 111a of the front connecting tube 111. Therefore, a gap 143 is formed between the hollow member 141 and a proximal end portion 111b of the front connecting tube 111. In an axial direction of a spiral tube 81, a length of the hollow member 141 is substantially the same as a length of the front connecting tube 111. The hollow member 141 is accommodated in the front connecting tube 111, and it does not protrude from the distal end portion 111a and the proximal end portion 111b of the front connecting tube 111.

A non-illustrated built-in matter such as an imaging cable or an operation wire configured to bend a bend portion 23 is inserted in the hollow member 141. The built-in matter is arranged from a distal end hard portion 21 to an operating portion 60 in an endoscope 1.

In the front connecting tube 111 and the hollow member 141 which is accommodated in the front connecting tube 111, a distal end portion 81a side is arranged between an outer peripheral surface of the hollow member 141 and an inner peripheral surface of the front connecting tube 111 in such a manner that the hollow member 141 is inserted into the distal end portion 81a side, the distal end portion 81a side is wound around the hollow member 141, and the distal end portion 81a side is arranged in the gap 143. The distal end portion 81a side can move on the outer peripheral surface of the hollow member 141 along the axial direction of the spiral tube 81 and moves in the gap 143.

In the front connecting tube 111, the hollow member 141 separates the non-illustrated built-in matter from the spiral tube 81. As a result, the hollow member 141 prevents the distal end portion 81a side and the built-in matter from interfering with each other when the distal end portion 81a side slides in the front connecting tube 111. In other words, the hollow member 141 prevents the built-in matter from becoming worn away by the sliding distal end portion 81a side and prevents the distal end portion 81a side from becoming worn away by the built-in matter when the distal end portion 81a slides.

As described above, in this embodiment, the hollow member 141 can prevent the mutual interference of the distal end portion 81a side and the built-in matter. Additionally, in this embodiment, the hollow member 141 can guide slide of the distal end portion 81a side.

It is to be noted that the description has been given as to the arrangement of the hollow embodiment 141 in the front connecting tube 111 in this embodiment, but the present invention is not restricted thereto, and the hollow member 141 may be arranged in the rear connecting tube 121 like the front connecting tube 111. That is, in this embodiment, the flexible tube portion 25 further has the hollow member 141 which is arranged in at least one of the front connecting tube 111 and the rear connecting tube 121. At this time, the hollow member 141 is narrower than the connecting tube in which the hollow member 141 is arranged. The end portion side that slides in the connecting tube in which the hollow member 141 is arranged is arranged between the outer peripheral surface of the hollow member 141 and the inner peripheral surface of the connecting tube in which the hollow member 141 is arranged so that the end portion side is wound around the hollow member 141. Further, the end portion side can move on the outer peripheral surface of the hollow member 141 along the axial direction of the spiral tube 81.

The present invention is not restricted to the foregoing embodiments as it is, and constituent elements can be modified and embodied without deviating from the gist on the embodying stage. Furthermore, appropriately combining constituent elements disclosed in the foregoing embodiments enables forming various kinds of inventions.

## Claims

1. A flexible tube portion (25) of an endoscope (1), comprising:
a spiral tube (81);
a first connecting tube (111) which is arranged on a distal end portion (81a) side of the spiral tube (81) and connected to a node ring (23b) of a bend portion (23) in the endoscope (1); and
a second connecting tube (121) which is arranged on a proximal end portion (81b) side of the spiral tube (81) and connected to an operating portion (60) in the endoscope (1),
wherein the spiral tube (81) is movable along an axial direction of the spiral tube (81) with respect to at least one of the first connecting tube (111) and the second connecting tube (121).

2. The flexible tube portion (25) of an endoscope (1) according to claim 1, further comprising:
a mesh tube (91); and a resin layer (101),
wherein both end portions of at least one of the mesh tube (91) and the resin layer (101) are fixed to the first connecting tube (111) and the second connecting tube (121), respectively.

3. The flexible tube portion (25) of an endoscope (1) according to claim 1,
wherein at least one of the distal end portion (81a) and the proximal end portion (81b) of the spiral tube (81) is fixed to the connecting tube through a second elastic portion (131) of the spiral tube (81) which stretches or contracts more than a first elastic portion (81c) of the spiral tube (81).

4. The flexible tube portion (25) of an endoscope (1) according to claim 1,
wherein at least one of the distal end portion (81a) and the proximal end portion (81b) of the spiral tube (81) is fixed to the connecting tube through an elastic member (133) which stretches or contracts more than the spiral tube (81).

5. The flexible tube portion (25) of an endoscope (1) according to claim 1,
wherein, when the spiral tube (81) moves in the axial direction of the spiral tube (81) with respect to the first connecting tube (111), this means that the distal end portion (81a) of the spiral tube (81) slides in the first connecting tube (111) in the axial direction of the spiral tube (81), and
when the spiral tube (81) moves in the axial direction of the spiral tube (81) with respect to the second connecting tube (121), this means that the proximal end portion (81b) of the spiral tube (81) slides in the second connecting tube (121) in the axial direction of the spiral tube (81).

6. The flexible tube portion (25) of an endoscope (1) according to claim 1,
wherein the spiral tube (81) is formed of a loose winding, a compact winding, or a close coil having initial tension given thereto.

7. The flexible tube portion (25) of an endoscope (1) according to claim 1,
wherein a length L2 of the spiral tube (81) from the distal end portion (81a) to the proximal end portion (81b) is longer than a length L3 from an insertion opening (113) of the first connecting tube (111) into which the distal end portion (81a) side is inserted to an insertion opening (123) of the second connecting tube (121) into which the proximal end portion (81b) is inserted.

8. The flexible tube portion (25) of an endoscope (1) according to claim 1, further comprising a hollow member (141) which is arranged in at least one of the first connecting tube (111) and the second connecting tube (121),
wherein the spiral tube (81) is movable on an outer peripheral surface of the hollow member (141) along the axial direction of the spiral tube (81).

9. An endoscope (1) comprising the flexible tube portion (25) of an endoscope (1) according to one of claims 1 to 8.
